# EUROPEAN PATENT SPECIFICATION

(11) **EP 1 750 741 B1**
(45) Date of publication and mention of the grant of the patent: **02.12.2015**
(21) Application number: 05779362.2
(22) Date of filing: 09.05.2005
(51) Int. Cl.: A61K 38/02, A61P 25/00

(54) **METHODS OF TREATING DISEASE WITH RANDOM COPOLYMERS**
VERFAHREN ZUR BEHANDLUNG VON KRANKHEITEN MIT STATISTISCHEN COPLYMEREN
METHODES DE TRAITEMENT DE MALADIES PAR COPOLYMERES ALEATOIRES

(30) Priority: 07.05.2004 US 569292 P; 18.03.2005 US 663333 P
(43) Date of publication of application: 14.02.2007
(60) Divisional application: 12174215.9
(73) Proprietor: ARES TRADING S.A., 1170 Aubonne (CH)
(72) Inventor: RASMUSSEN, James, Cambridge, MA 02142 (US); ZHANG, Jianxin, Acton, Massachusetts 01720 (US); BALDWIN, Sam, Westford, Massachusetts 01886 (US); ZANELLI, Eric, Sudbury, Massachusetts 01776 (US); YU, Bei, West Roxbury, Massachusetts 02132 (US); BONNIN, Dustan, Belmont, Massachusetts 02478 (US); JOHNSON, Keith, Lincoln MA 01773-2605 (US)
(74) Representative: Potter Clarkson LLP
(86) International application number: PCT/US2005/016344
(87) International publication number: WO 2005/112972

(56) References cited:
- WO-A-03/029276
- WO-A-03/047500
- FRIDKIS-HARELI MASHA ET AL: "Novel synthetic amino acid copolymers that inhibit autoantigen-specific T cell responses and suppress experimental autoimmune encephalomyelitis." THE JOURNAL OF CLINICAL INVESTIGATION. JUN 2002, vol. 109, no. 12, June 2002 (2002-06), pages 1635-1643, XP002311466 ISSN: 0021-9738

## Description

### CROSS-REFERENCE TO RELATED APPLICATIONS

This application claims priority to U.S. Provisional Application Ser. No. 60/569292 filed May 7, 2004, and to U.S. Provisional Application Ser. No. 60/663333 filed March 18, 2005.

### BACKGROUND OF THE INVENTION

An autoimmune disease results from an inappropriate immune response directed against a self antigen (an autoantigen), which is a deviation from the normal state of self-tolerance. Self-tolerance arises when the production of T cells and B cells capable of reacting against autoantigens has been prevented by events that occur in the early development of the immune system. The cell surface proteins that play a central role in regulation of immune responses through their ability to bind and present processed peptides to T cells are the major histocompatibility complex (MHC) molecules (Rothbard, J.B., et al., 1991, Annu. Rev. Immunol. 9:527). Autoimmune diseases include rheumatoid arthritis (RA), multiple sclerosis (MS), human type I or insulin-dependent diabetes mellitus (IDDM), autoimmune uveitis, primary biliary cirrhosis (PBC) and celiac disease.

One target for inhibition of an autoimmune response is the set of lymphocyte surface protein MHC molecules, particularly a protein encoded by an MHC class II gene, for example, HLA-DR, -DQ and -DP. Each of the MHC genes is found in a large number of alternative or allelic forms within a mammalian population. The genomes of subjects affected with certain autoimmune diseases, for example MS and RA, are more likely to carry one or more characteristic MHC class II alleles, to which that disease is linked.

A number of therapeutic agents have been developed to treat autoimmune diseases, including general anti-inflammatory drugs such as COX-2 inhibitors, *i.e.,* agents that can prevent formation of low molecular weight inflammatory compounds by inhibiting a cyclooxygenase; agents that can function by inhibiting a protein mediator of inflammation, for example, by sequestering the inflammatory protein tumor necrosis factor (TNF) with an anti-TNF specific monoclonal antibody or antibody fragment, or with a soluble form of the TNF receptor; and agents that target a protein on the surface of a T cell and generally prevent interaction with an antigen presenting cell (APC) by inhibiting the CD4 receptor or the cell adhesion receptor ICAM-1. However, compositions having natural folded proteins as therapeutic agents can encounter problems in production, formulation, storage, and delivery. Several of these problems necessitate delivery to the patient in a hospital setting.

An agent that interacts and binds relatively nonspecifically to several MHC class II molecules is Copolymer 1 (Cop 1), a synthetic amino acid heteropolymer that was shown to be capable of suppressing experimental allergic encephalomyelitis (EAE; Sela, M. et al., 1990, Bull. Inst. Pasteur (Paris)), which can be induced in the mouse and is a model for MS. Copolymer 1, which is poly(Y,E,A,K) also known as glatiramer acetate or "YEAK" using the one letter amino acid code (see infra; Y represents tyrosine, E glutamic acid, A alanine, and K lysine), has been used to treat relapsing forms of MS but does not suppress the disease entirely (Bornstein, M.B., et al, 1987, N. Engl. J. Med. 317:408; Johnson, K.P. et al., 1995, Neurology 45:1268).

Although random copolymers may be effective for the treatment of autoimmune diseases (Simpson, D. et al., 2003, Biodrugs 17(3):207-10), their repeated administration may cause undesired side effects. Accordingly, there is a need for improved methods for the treatment of autoimmune diseases with random copolymers which result in fewer side effects.

WO 03/029276 discloses specified copolymers for the suppression of autoimmune diseases.

### BRIEF SUMMARY OF THE INVENTION

The subject matter of the invention is set out in the appended claims.

### BRIEF DESCRIPTION OF THE DRAWINGS

Figure 1 shows the effect of copolymer administration on the disease progression of EAE.
Figure 2. shows the survival rate of mice with EAE when administered with random copolymers.
Figure 3 shows IgG antibody production against copolymers administered at daily or weekly doses.
Figure 4 shows IgG1 antibody production against copolymers administered at daily or weekly doses.
Figure 5 shows IgG2b antibody production against copolymers administered at daily or weekly doses.
Figure 6 shows the changes in antibody titer against copolymers during the time course of a treatment.
Figure 7 shows the IgG1 antibody production against PLP peptide in mice administered with random copolymers.
Figure 8 shows the IgG2b antibody production against PLP peptide in mice administered with random copolymers.
Figure 9 shows the ratio of IL-13 over IFN γ in mice administered with random copolymers.
Figure 10 shows the bias for induction of TH2 related cytokines compared to TH1 related cytokines in mice administered with random copolymers.

### DETAILED DESCRIPTION OF THE INVENTION

### I. Overview

In the invention, the disease is mediated by TH1 cells. The disease is an autoimmune disease. In one embodiment, the disease is selected from the group consisting of multiple sclerosis, type-I diabetes, Hashimoto's thyroiditis, Crohn's disease, rheumatoid arthritis, systemic lupus erythematosus (SLE), gastritis, autoimmune hepatitis, hemolytic anemia, autoimmune hemophilia, autoimmune lymphoproliferative syndrome (ALPS), autoimmune uveoretinitis, glomerulonephritis, Guillain-Barré syndrome, psoriasis, myasthenia gravis, autoimmune encephalomyelitis, Goodpasture's syndrome, Grave's disease, paraneoplastic pemphigus, autoimmune thrombocytopenic purpura, scleroderma with anti-collagen antibodies, mixed connective tissue disease, pernicious anemia, polymyositis, idiopathic Addison's disease, autoimmune-associated infertility, glomerulonephritis, bullous pemphigoid, Sjogren's syndrome, idiopathic myxedema and colitis. In preferred embodiments, the disease is multiple sclerosis or relapsing-remitting multiple sclerosis. In preferred embodiments of the invention, the subject is a mammal, or more preferably a human.

In the invention, the dosing regimen comprises subcutaneous administration. The random copolymer may also be administered via devices designed to deliver the random copolymer continuously, such as a transdermal patch or pump or implant. For example, a transdermal patch may be used to administer the random copolymer over a span of 12 hours every 48 hours or longer. In a related aspect, the copolymer is administered in a sustained release formulation.

In preferred embodiments, the sustained release formulation administers the copolymer over a period of at least 2, 3, 4, 5, 6, 7, 8, 9, 10, 11, 12, 13 or 14 days. In another embodiment, the total dosage delivered daily by the sustained release formulation is less than 90%, 80%, 70%, 60%, 50%, 40%, 30%, 20%, 10% or 5% of a daily dosage known to be effective in the treatment of the disease. In a specific embodiment, the sustained release formulation administers 25% or less, per day, of a dosage of a random copolymer which is known to be effective in treating the disease when administered daily.

In some embodiments of the invention described herein, the use further comprises administering an additional therapeutically active agent to the subject, such as an anti-inflammatory agent. In preferred embodiments, the agent is useful in treating the disease. In another preferred embodiment, the agent synergizes with the random copolymer to treat the disease.

In the invention described herein, the dosing regimen comprises administering the random copolymer to the subject multiple times, with a time interval between each administration. The time interval between each administration is at least 7 days.

In some embodiments of the invention described herein, the effective amount of the random copolymer is between 0.02 mg per dose and 2000 mg per dose, or more preferably between 2 mg per dose and 200 mg per dose.

### II. Definitions

For convenience, certain terms employed in the specification, examples, and appended claims, are collected here. Unless defined otherwise, all technical and scientific terms used herein have the same meaning as commonly understood by one of ordinary skill in the art to which this invention belongs.

The articles "a" and "an" are used herein to refer to one or to more than one (i.e., to at least one) of the grammatical object of the article. By way of example, "an element" means one element or more than one element.

The term "including" is used herein to mean, and is used interchangeably with, the phrase "including but not limited" to.

The term "or" is used herein to mean, and is used interchangeably with, the term "and/or," unless context clearly indicates otherwise.

The term "such as" is used herein to mean, and is used interchangeably, with the phrase "such as but not limited to".

A "patient" or "subject" to be treated by the method of the invention can mean either a human or non-human animal, preferably a mammal.

The term "autoimmune condition" or "autoimmune disease" means a disease state caused by an inappropriate immune response that is directed to a self-encoded entity which is known as an autoantigen. The copolymer compounds provided herein can be used to treat symptoms of an autoimmune disease, a class of disorder which includes Hashimoto's thyroiditis; idiopathic myxedema, a severe hypothyroidism; multiple sclerosis, a demyelinating disease marked by patches or hardened tissue in the brain or the spinal cord; myasthenia gravis which is a disease having progressive weakness of muscles caused by autoimmune attack on acetylcholine receptors at neuromuscular junctions; Guillain-Barre syndrome, a polyneuritis; systemic lupus erythematosis; uveitis; autoimmune oophoritis; chronic immune thrombocytopenic purpura; colitis; diabetes; Grave's disease, which is a form of hypothyroidism; psoriasis; pemphigus vulgaris; and rheumatoid arthritis (RA).

The term "demyelinating condition" includes a disease state in which a portion of the myelin sheath, consisting of plasma membrane wrapped around the elongated portion of the nerve cell, is removed by degradation. A demyelinating condition can arise post-vaccination, post-anti TNF treatment, post-viral infection, and in MS.

The term "derivative" of an amino acid means a chemically related form of that amino acid having an additional substituent, for example, N-carboxyanhydride group, a γ-benzyl group, an ε-N-trifluoroacetyl group, or a halide group attached to an atom of the amino acid.

The term "analog" means a chemically related form of that amino acid having a different configuration, for example, an isomer, or a D-configuration rather than an L-configuration, or an organic molecule with the approximate size, charge, and shape of the amino acid, or an amino acid with modification to the atoms that are involved in the peptide bond, so that the copolymer having the analog residue is more protease resistant than an otherwise similar copolymer lacking such analog, whether the analog is interior or is located at a terminus of the copolymer, compared to the copolymer without the analog.

The phrases "amino acid" and "amino acid copolymer" can include one or more components which are amino acid derivatives and/or amino acid analogs as defined herein, the derivative or analog comprising part or the entirety of the residues for any one or more of the 20 naturally occurring amino acids indicated by that composition. For example, in an amino acid copolymer composition having one or more tyrosine residues, a portion of one or more of those residues can be substituted with homotyrosine. Further, an amino acid copolymer having one or more non-peptide or peptidomimetic bonds between two adjacent residues is included within this defmition.

The term "hydrophobic" amino acid means aliphatic amino acids alanine (A, or ala), glycine (G, or gly), isoleucine (I, or ile), leucine (L, or leu), methionine (M, or met), proline (P, or pro), and valine (V, or val), the terms in parentheses being the one letter and three letter standard code abbreviations for each amino acid, and aromatic amino acids tryptophan (W, or trp), phenylalanine (F, or phe), and tyrosine (Y, or tyr). These amino acids confer hydrophobicity as a function of the length of aliphatic and size of aromatic side chains, when found as residues within a copolymer or other polypeptide.

The term "charged" amino acid means amino acids aspartic acid (D or asp), glutamic acid (E or glu), arginine (R or arg) and lysine (K or lys), which confer a positive (lys, and arg) or negative (asp, glu) charge at physiological values of pH on an aqueous solution of a copolymer or other amino acid composition containing one or more residues of these amino acids. Histidine (H or his) is hydrophobic at pH 7, and charged at pH 6.

The terms "disorders" and "diseases" are used inclusively and refer to any deviation from the normal structure or function of any part, organ or system of the body (or any combination thereof). A specific disease is manifested by characteristic symptoms and signs, including biological, chemical and physical changes, and is often associated with a variety of other factors including, but not limited to, demographic, environmental, employment, genetic and medically historical factors. Certain characteristic signs, symptoms, and related factors can be quantitated through a variety of methods to yield important diagnostic information.

The term "prophylactic" or "therapeutic" treatment refers to administration to the subject of one or more of the subject compositions. If it is administered prior to clinical manifestation of the unwanted condition (*e.g*., disease or other unwanted state of the host animal) then the treatment is prophylactic, *i.e.,* it contributes to protection of the host against developing the unwanted condition, whereas if administered after manifestation of the unwanted condition, the treatment is therapeutic (*i*.*e.,* it is intended to diminish, ameliorate or prevent progression of the unwanted condition or side effects therefrom).

The term "therapeutic effect" refers to a local or systemic effect in animals, particularly mammals, and more particularly humans, caused by a pharmacologically active substance. The term thus means any substance intended for use in the diagnosis, cure, mitigation, treatment or prevention of disease or in the enhancement of desirable physical or mental development and conditions in an animal or human. The phrase "therapeutically-effective amount" means that amount of such a substance that produces some desired local or systemic effect at a reasonable benefit/risk ratio applicable to any treatment. In certain embodiments, a therapeutically-effective amount of a compound will depend on its therapeutic index, solubility, and the like. For example, certain compounds discovered by the methods of the present invention may be administered in a sufficient amount to produce a reasonable benefit/risk ratio applicable to such treatment.

The term "effective amount" refers to the amount of a therapeutic reagent that when administered to a subject by an appropriate dose and regimen produces the desired result.

The term "subject in need of treatment for a disorder" is a subject diagnosed with that disorder, likely to develop the disorder, or is suspected of having that disorder.

The term "antibody" as used herein is intended to include whole antibodies, e.g., of any isotype (IgG, IgA, IgM, IgE, etc), and includes fragments thereof which are also specifically reactive with a vertebrate, e.g., mammalian, protein. Antibodies can be fragmented using conventional techniques and the fragments screened for utility and/or interaction with a specific epitope of interest. Thus, the term includes segments of proteolytically-cleaved or recombinantly-prepared portions of an antibody molecule that are capable of selectively reacting with a certain protein. Non-limiting examples of such proteolytic and/or recombinant fragments include Fab, F(ab')2, Fab', Fv, and single chain antibodies (scFv) containing a V[L] and/or V[H] domain joined by a peptide linker. The scFv's may be covalently or non-covalently linked to form antibodies having two or more binding sites. The term antibody also includes polyclonal, monoclonal, or other purified preparations of antibodies and recombinant antibodies.

The term "central tolerance" means tolerance for an antigen controlled by the events in the thymus, namely the clonal deletion of T cells reactive to the antigen in the thymus gland. Partially activated T cells with high affinity receptors for the antigen undergo negative selection and clonal deletion in the thymus by Fas-mediated apoptosis, triggered by coexpression and binding of FasL to Fas on the cell surface. In contrast, the term "peripheral tolerance" means deletion of T cells by activation-induced cell death (AICD) and functional silencing (clonal anergy) of T cells without clonal deletion in the spleen. Also, when lacking the cooperation of helper T cells, B cells are presumably "helpless" to respond to T cell dependent antigens. The modulation of central and peripheral tolerance is regulated by phosphorylation of p56 ^{lck} and ZAP-70. The status and the degree of phosphorylation of key residues of these proteins result in up or down regulation of signaling molecules that influence the peripheral and central tolerance. Inhibition of T cell receptor signaling also plays a role in inducing tolerance.

Other technical terms used herein have their ordinary meaning in the art that they are used, as exemplified by a variety of technical dictionaries.

### III. Random Copolymers

The composition of a random copolymer for use in the present invention comprises the characteristics of a compilation of a multiplicity of cross-reactive T cell epitopes. The composition of a random copolymer of the instant invention may further comprise the characteristics of altered peptide ligands. Multiple functional consequences of the composition of a random copolymer of the instant invention exist: one is the potential to functionally interact with thousands, preferably hundreds of thousands, more preferably millions, of T cell epitopes via presentation by MHC molecules, preferably MHC class II molecules, while another is the generation of random copolymer specific T cells which may secrete soluble mediators, such as cytokines.

A random copolymer for use in the present invention may be given specific amino acid sequence characteristics such that the selected sub-group of amino acids preferentially interacts with specific T cell epitopes, some of which may be directly associated with pathogenic disorders.

### Copolymers comprising four amino acids

In the invention, the random copolymer consists of amino acid residues YFAK (L-tyrosine, L-phenylalanine, L-alanine and L-lysine) in a molar output ratio of about 1.0:1.2:X_{A}:6.0 respectively, wherein X_{A} is greater than 20.0 and less than 30.0, and the variability in the output ratios comprises a range of about 10% between the different amino acids. The molar output ratios of YFAK of random copolymers of preferred embodiments are shown in Table I below:

**Table I: Amino Acid Composition Ratios of Random Copolymers**

| Y | F | A | K |
|---|---|---|---|
| 1.0: | 1.2: | 20.0: | 6.0 |
| 1.0: | 1.2: | 22.0: | 6.0 |
| 1.0: | 1.2: | 24.0: | 6.0 |

The length of any of such copolymer is between 35 and 75 amino acids residues. More preferably, the length of a random copolymer is between 35 and 65 amino acid residues. In a preferred embodiment the length of a random copolymer is about 50 amino acids. In another preferred embodiment, the length of a random copolymer is about 52 amino acids.

In a preferred embodiment, the average molar output ratio of YFAK is about 1.0: 1.2: X_{A}:6.0, wherein X_{A} is greater than 20, and the ratio of alanine increases with the length of copolymer. In a preferred embodiment, the length of such random copolymer is about 52 amino acid residues, and the ratio of alanine composition in amino acid positions 31-52 is greater than in amino acid positions 11-30, and the ratio of alanine composition in amino acid positions 11-30 is greater than in amino acid positions 1-10.

### Copolymers that bind to MHC class II proteins

In one embodiment, the copolymers used in the invention described herein are capable of binding to an MHC class II protein which is associated with an autoimmune disease. There are at least three types of Class II MHC molecules: HLA-DR, HLA-DQ, and HLA-DP molecules. There are also numerous alleles encoding each type of these HLA molecules. The Class II MHC molecules are expressed predominantly on the surfaces of B lymphocytes and antigen presenting cells such as macrophages. Any available method can be used to ascertain whether the copolymer binds to one or more MHC class II proteins. For example, the polypeptide can be labeled with a reporter molecule (such as a radionuclide or biotin), mixed with a crude or pure preparation of MHC class II protein and binding is detected if the reporter molecule adheres to the MHC class II protein after removal of the unbound polypeptide.

In another embodiment, the copolymers used in the invention described herein are capable of binding to an MHC class II protein associated with multiple sclerosis. A polypeptide of this embodiment can have similar or greater affinity for the antigen binding groove of an MHC class II protein associated with multiple sclerosis than does Copolymer 1. Hence, the contemplated polypeptide can inhibit binding of or displace the binding of myelin autoantigens from the MHC class II protein. One MHC class II protein associated with multiple sclerosis is HLA-DR4 (DRB1*1501).

In another embodiment, the random copolymers used in the invention described herein are capable of binding to an MHC class II protein associated with an arthritic condition, for example, rheumatoid arthritis or osteoarthritis. A random copolymer of this embodiment can have a greater affinity for the antigen binding groove of an MHC class II protein associated with the autoimmune disease than does a type II collagen 261-273 peptide. Hence, the contemplated random copolymer described herein can inhibit binding of or displace the type II collagen 261-273 peptide from the antigen binding groove of an MHC class II protein. The Class II MHC protein consists of approximately equal-sized alpha and beta subunits, both of which are transmembrane proteins. A peptide-binding cleft is formed by parts of the amino termini of both α and β subunits. This peptide-binding cleft is the site of presentation of the antigen to T cells.

In other embodiments, the random copolymers used in the invention can bind to the peptide binding groove of the HLA-DR molecules. As binding motifs of Cop 1 to MS-associated HLA-DR molecules are known (Fridkis-Hareli et al, 1999, J. Immunol.; 162(8):4697-704), polypeptides of fixed sequence can readily be prepared and tested for binding to the peptide binding groove of the HLA-DR molecules as described in Fridkis-Hareli. Examples of such peptides are those disclosed in WO 00/005249. Thirty-two of the peptides specifically disclosed in said application are as follows:

AAAYAAAAAAKAAAA;  AEKYAAAAAAKAAAA;  AKEYAAAAAAKAAAA;
AKKYAAAAAAKAAAA;  AEAYAAAAAAKAAAA;  KEAYAAAAAAKAAAA;
AEEYAAAAAAKAAAA;  AAEYAAAAAAKAAAA;  EKAYAAAAAAKAAAA;
AAKYEAAAAAKAAAA   AAKYAEAAAAKAAAA;  EAAYAAAAAAKAAAA;
EKKYAAAAAAKAAAA;  EAKYAAAAAAKAAAA;  AEKYAAAAAAAAAAA;
AKEYAAAAAAAAAAA;  AKKYEAAAAAAAAAA;  AKKYAEAAAAAAAAA;
AEAYKAAAAAAAAAA;  KEAYAAAAAAAAAAA;  AEEYKAAAAAAAAAA;
AAEYKAAAAAAAAAA;  EKAYAAAAAAAAAAA;  AAKYEAAAAAAAAAA;
AAKYAEAAAAAAAAA;  EKKYAAAAAAAAAAA;  EAKYAAAAAAAAAAA;
AEYAKAAAAAAAAAA;  AEKAYAAAAAAAAAA;  EKYAAAAAAAAAAAA;
AYKAEAAAAAAAAAA;  AKYAEAAAAAAAAAA.

Additional random copolymers for use in the present invention, and methods of synthesizing them, may be found in the literature, such as in Shukaliak Quandt, J. et al., 2004, Mol. Immunol. 40(14-15):1075-87; Montaudo, M.S., 2004, J. Am. Soc. Mass Spectrom. 15(3):374-84; Takeda, N. et al., 2004, J Control Release 95(2):343-55; Pollino, J.M. et al., 2004, J. Am. Chem. Soc. 126(2):563-7; Fridkis-Hareli, M. et al., 2002, J. Clin Invest. 109(12):1635-43; Williams, D.M. et al., 2000, J. Biol. Chem. 275(49):38127-30; Tselios, T. et al., 2000, Bioorg. Med Chem. 8(8): 1903-9; and Cady, C.T. et al., 2000, J Immunol. 165(4): 1790-8.

In certain preferred embodiments, the copolymers of the invention bind to HLA-DQA1 molecules, and in even more preferably to one or more of HLA molecules encoded in the alleles DQA1*0501-DQB1*0201, DQA1*0301, DQB1*0401, and DQA1*03-DQB1*0302.

In other embodiments, the copolymers of the present invention bind to certain HLA-DQ molecules that predispose the carrier of such molecules to autoimmune-associated diseases, such as type I diabetes and celiac disease, with a dissociation constant (K_{d}) at least 10 times less than the copolymer's K_{d} for binding HLA-DR molecules and/or other DQ isotypes. Such HLA-DQ molecules are the combined protein products of specific HLA-DQB1 and DQA1 alleles known as DQB1*0201, DQB1*0302, DQB1*0304, DQB1*0401, DQB1*0501, DQB1*0502; and DQA1*0301, DQA1*0302, DQA1*0303, DQA1*0501. These alleles may be encoded on the same haplotypes ("cis" alleles) such as DQB 1*0201-DQA1*0501-DRB1*0301 and DQB1*0302-DQA1*0301-DRB1*0401. The resulting HLA molecule comprising polypeptide products of "cis" alleles are herein referred to as "cis dimer." Alternatively, the alleles may be encoded on different haplotypes ("trans" alleles). The HLA molecule comprising polypeptide products of "trans" alleles are herein referred to as "trans" dimer. An example of "trans" alleles is the combination of DQB1*0201 on DQB1*0201-DQA1*0501-DRB1*0301 and DQA1*0301 on DQB1*0301-DQA1*0301-DRB1*0404.

In preferred embodiments, the copolymers compositions of the present invention bind to one or more DQ isotypes with an average K_{d} of 1µM or less, and more preferably an average K_{d} less than 100nM, 10nM or even 1nM. Another way to identify preferred copolymers is based on the measure of a copolymer to displace another in competitive binding assays, such as described in Sidney et al., 2002, J. Immunol. 169:5098, which is expressed as an IC₅₀ value. Preferred copolymers of the present invention have IC₅₀'s less than 1µM, more preferably less than 500nM, and even more less than 100nM.

In certain preferred embodiments, the copolymer is formed by random synthesis (polymerization) of the various amino acid residues.

When synthesized, a typical preparation of random copolymers is a mixture of peptides of various lengths, the majority of which are of the desired length but containing shorter or longer peptides inevitably created by the currently available synthetic processes.

In certain preferred embodiments, the subject copolymers are formulated for use as a medicament so as to have a polydispersity less than 25,000, and more preferably less than 10000, 5000, 1000, 500, 100, 50, or even less than 10.

### Synthesis of random copolymers

The random copolymers used in the present invention can be made by any procedure available to one of skill in the art.

For purposes of this application, the terms "ambient temperature" and "room temperature" mean a temperature ranging from about 20 to about 26°C.

A preferred synthesis method of the random copolymers of the present invention is by solid phase synthesis. The synthesis is done in multiple steps by the Solid Phase Peptide Synthesis (SPPS) approach using Fmoc protected amino acids. SPPS is based on sequential addition of protected amino acid derivatives, with side chain protection where appropriate, to a polymeric support (bead). The base-labile Fmoc group is used for N-protection. After removing the protecting group (via piperidine hydrolysis) the next amino acid mixture is added using a coupling reagent (TBTU). After the final amino acid is coupled, the N-terminus is acetylated.

The resulting peptide (attached to the polymeric support through its C-terminus) is cleaved with TFA to yield the crude peptide. During this cleavage step, all of the side chains protecting groups are also cleaved. After precipitation with diisopropyl ether, the solid is filtered and dried. The resulting peptide is analyzed and stored at 2-8°C.

### Example of Solid Phase Synthesis

The random copolymer YFAK consisting of L-alanine, L-lysine, L-phenylalanine and L-tyrosine is prepared in its protected form on Wang resin. Resins used were Fmoc-L-Tyr(t-Bu)-Wang (0.62 mmol/g), Fmoc-L-Phe-Wang (0.72 mmol/g), Fmoc-L-Ala-Wang (0.70 mmol/g), and Fmoc-L-Lys(Boc)-Wang (0.72 mmol/g). The four F-moc protected amino acids, Fmoc-L-Tyr(t-Bu)-OH, Fmoc-L-Phe-OH, Fmoc-L-Ala-OH, and Fmoc-L-Lys-OH, are used in a molar input ratio of 1:1:10:6 respectively during each coupling step. Other reagents used in the synthesis are 2-(1H-Benzotriazole-1-yl)-1,1,3,3-tetramethyluronium, tetrafluoroborate (TBTU), N,N-diisopropylethylamine (DIPEA), piperidine, and trifluoroacetic acid (TFA). The solvents used are N-methylpyrrolidone (NMP), isopropanol (IsOH, IPA, i-PrOH), methylene chloride, and isopropyl ether. The stoichiometry of each coupling is as follows:
- residues 1 through 10 using 2 equivalents of Fmoc protected amino acids;
- residues 11 through 30 using 2 equivalents with double coupling of Fmoc protected amino acids;
- residues 31 through 52 using 2.5 equivalents of Fmoc protected amino acids with double coupling.

An example of amino acid input ratios in a representative example of YFAK synthesis with progressively higher alanine contents is as follows:

| Positions | Y | F | A | K |
|---|---|---|---|---|
| 0-10 | 3.7 | 5.5 | 64.4 | 26.4 |
| 11-20 | 4.3 | 5.1 | 71.4 | 19.2 |
| 21-30 | 4.0 | 4.7 | 71.5 | 19.8 |
| 31-40 | 3.6 | 4.7 | 74.3 | 17.4 |
| 41-52 | 3.0 | 4.1 | 76.0 | 16.8 |

### Unnatural polypeptides and chemical modification of copolymers

The molecular weight of a random copolymer can be adjusted during polypeptide synthesis or after the copolymer have been synthesized. To adjust the molecular weight during polypeptide synthesis, the synthetic conditions or the amounts of amino acids are adjusted so that synthesis stops when the polypeptide reaches the approximate length which is desired. After synthesis, polypeptides with the desired molecular weight can be obtained by any available size selection procedure, such as chromatography of the polypeptides on a molecular weight sizing column or gel, and collection of the molecular weight ranges desired. The present polypeptides can also be partially hydrolyzed to remove high molecular weight species, for example, by acid or enzymatic hydrolysis, and then purified to remove the acid or enzymes.

In one embodiment, the random copolymers with a desired molecular weight may be prepared by a process which includes reacting a protected polypeptide with hydrobromic acid to form a trifluoroacetyl-polypeptide having the desired molecular weight profile. The reaction is performed for a time and at a temperature which is predetermined by one or more test reactions. During the test reaction, the time and temperature are varied and the molecular weight range of a given batch of test polypeptides is determined. The test conditions which provide the optimal molecular weight range for that batch of polypeptides are used for the batch. Thus, a trifluoroacetyl-polypeptide having the desired molecular weight profile can be produced by a process which includes reacting the protected polypeptide with hydrobromic acid for a time and at a temperature predetermined by test reaction. The trifluoroacetyl-polypeptide with the desired molecular weight profile is then further treated with an aqueous piperidine solution to form a low toxicity polypeptide having the desired molecular weight.

In one preferred embodiment, a test sample of protected polypeptide from a given batch is reacted with hydrobromic acid for about 10-50 hours at a temperature of about 20-28°C. The best conditions for that batch are determined by running several test reactions. For example, in one embodiment, the protected polypeptide is reacted with hydrobromic acid for about 17 hours at a temperature of about 26°C.

International PCT Publication Nos. WO 00/05250, WO 00/05249; WO 02/59143, WO 0027417, WO 96/32119, U.S. Patent Publication Nos. 2004/003888, 2002/005546, 2003/0004099, 2003/0064915 and 2002/0037848, U.S. Pat. Nos. 6,514,938, 5,800,808 and 5,858, 964, and PCT application PCT/US05/06822 further describe methods of synthesizing random copolymers, compositions comprising random copolymers, therapeutic formulations of random copolymers, methods of administering random copolymers to a subject, diseases that may be treated with random copolymers, and additional therapeutically effective agents which may be co-administered to a subject in with the random copolymers.

It is clear that this is given by way of example only, and that the composition can be varied both with respect to the constituents and relative proportions of the constituents if the above general criteria are adhered to.

### IV. Diseases

The invention provides compositions for treating or preventing diseases in a subject. A subject who is at risk of developing a disease, who is suspected of being afflicted with a disease, or who is afflicted with the disease may be treated using the compositions provided by the invention.

The disease that may be treated with the compositions for use of the present invention comprises a disease that is mediated by T_{H}1 cells.

The disease that may be treated with the compositions for use of the present invention comprises autoimmune diseases. Autoimmune diseases contemplated by the present invention include cell-mediated disease (*e.g*., T-cell). Such disorders can be inter alia arthritic conditions, demyelinating diseases and inflammatory diseases. The compositions of the invention are of particular interest for the treatment of demyelinating inflammatory diseases, which include multiple sclerosis, EAE, optic neuritis, acute transverse myelitis, and acute disseminated encephalitis. In one specific embodiment, any autoimmune disease can be treated by the present polypeptides so long as the contemplated polypeptide binds to an MHC class II protein that has been associated with the autoimmune disease. Progression of disease can be measured by monitoring clinical or diagnostic symptoms using known methods.

In one embodiment, the disease treated by the methods provided herein is an "arthritic condition". As used herein, an arthritic condition is a condition wherein at least one symptom of rheumatoid arthritis is observed in at least one joint of a mammal, for example in a shoulder, knee, hip, backbone or a digit of the mammal. RA is a common human autoimmune disease with a prevalence of about 1% among Caucasians (Harris, B. J. et al., 1997, In Textbook of Rheumatology 898-932), currently affecting 2.5 million Americans. RA is characterized by chronic inflammation of the synovial joints and infiltration by activated T cells, macrophages and plasma cells, leading to a progressive destruction of the articular cartilage. It is the most severe form of joint disease. Inherited susceptibility to RA is strongly associated with the affected subject having at the MHC class II DRB1 locus the allele DRB 1*0401, DRB 1*0404, or DRB1*0405 or the DRB1*0101 allele. The nature of the autoantigen(s) in RA is poorly understood, although collagen type II (CII) is a prominent candidate. An immunodominant T cell epitope in collagen type II corresponding to residues 261-273 has been identified (Fugger, L. et al., 1996, Eur. J Immunol. 26: 928-933).

Other examples of arthritic conditions include "polyarthritis", which is an arthritic condition that affects more than a single joint; "juvenile arthritis", an arthritic condition of humans under the age of 21; and Felty's syndrome, which can include the symptoms of neutropenia, splenomegaly, weight loss, anemia, lymphadenopathy, and pigment spots on the skin.

In another embodiment, the disease treated by the compositions provided herein is multiple sclerosis (MS). The course of disease for multiple sclerosis is highly varied, unpredictable, and, in most patients, remittent. The pathologic hallmark of MS is multicentric, multiphasic CNS inflammation and demyelination. Months or years of remission may separate episodes, particularly early in the disease. About 70% of patients of relapsing-remitting (RR) type, which is characterized by acute exacerbations with full or partial remissions. The remaining patients present with chronic progressive MS, which is subdivided further into (a) primary-progressive (PP), (b) relapsing-progressive (RP), which is a pattern combining features of RR and RP and is intermediate in clinical severity, and (c) secondary-progressive (SP), which many patients with RR progress to over time. In a specific preferred embodiment, the diseased treated by the present method is relapsing-remitting multiple sclerosis.

Clinical symptoms of MS include sensory loss (paresthesias), motor (muscle cramping secondary to spasticity) and autonomic (bladder, bowel, sexual dysfunction) spinal cord symptoms; cerebellar symptoms (e.g., Charcot triad of dysarthna, ataxia, tremor); fatigue and dizziness; impairment in information processing on neuropsychological testing; eye symptoms, including diplopia on lateral gaze; trigeminal neuralgia; and optic neuritis.

The autoantigen in MS most likely is one of several myelin proteins (e.g, proteolipid protein (PLP); myelin oligodendrocyte glycoprotein (MOG); myelin basic protein (MBP); myelin-associated glycoprotein (MAG), myelin-associated oligodendrocytic basic protein (MBOP); citrulline-modified MBP (the C8 isoform of MBP in which 6 arginines have been de-iminated to citrulline), cyclic nucleotide phosphodiesterase (CNPase), alpha-B crystalline, etc.) The integral membrane protein PLP is a dominant autoantigen of myelin. Microglial cells and macrophages perform jointly as antigen-presenting cells, resulting in activation of cytokines, complement, and other modulators of the inflammatory process, targeting specific oligodendroglia cells and their membrane myelin. A quantitative increase in myelin-autoreactive T_{H}1 cells with the capacity to secrete IFN-γ is associated with the pathogenesis of MS and EAE, suggesting that autoimmune inducer/helper T lymphocytes in the peripheral blood of MS patients may initiate and/or regulate the demyelination process in patients with MS. On the other hand, there is an extended literature on the protective role of TH2 cells producing anti-inflammatory cytokines such as IL-4 and IL-10. The shift of balance from T_{H}1 to T_{H}2 type of cells are expected to be beneficial to the prevention and treatment of MS and EAE.

In another embodiment, the disease treated by the invention provided herein is Insulin Dependent Diabetes Mellitus. Human type I or insulin-dependent diabetes mellitus (IDDM) is characterized by autoimmune destruction of the cells in the pancreatic islets of Langerhans. The depletion of β-cells results in an inability to regulate levels of glucose in the blood. Overt diabetes occurs when the level of glucose in the blood rises above a specific level, usually about 250 mg/dl. In humans a long pre-symptomatic period precedes the onset of diabetes. During this period there is a gradual loss of pancreatic beta cell function. The development of disease is implicated by the presence of autoantibodies against insulin, glutamic acid decarboxylase, and the tyrosine phosphatase IA2 (IA2), each an example of a self-protein, -polypeptide or -peptide according to this invention. Human IDDM is currently treated by monitoring blood glucose levels to guide injection, or pump-based delivery, of recombinant insulin. Diet and exercise regimens contribute to achieving adequate blood glucose control.

Markers that may be evaluated during the pre-symptomatic stage are the presence of insulitis in the pancreas, the level and frequency of islet cell antibodies, islet cell surface antibodies, aberrant expression of Class II MHC molecules on pancreatic beta cells, glucose concentration in the blood, and the plasma concentration of insulin. An increase in the number of T lymphocytes in the pancreas, islet cell antibodies and blood glucose is indicative of the disease, as is a decrease in insulin concentration.

The presence of combinations of autoantibodies with various specificities in serum is highly sensitive and specific for human type I diabetes mellitus. For example, the presence of autoantibodies against GAD and/or IA-2 is approximately 98% sensitive and 99% specific for identifying type I diabetes mellitus from control serum. In non-diabetic first degree relatives of type I diabetes patients, the presence of autoantibodies specific for two of the three autoantigens including GAD, insulin and IA-2 conveys a positive predictive value of >90% for development of type I DM within 5 years.

In another embodiment, the disease treated by the invention provided herein is autoimmune uveitis. Autoimmune uveitis is an autoimmune disease of the eye that is estimated to affect 400,000 people, with an incidence of 43,000 new cases per year in the U.S. Autoimmune uveitis is currently treated with steroids, immunosuppressive agents such as methotrexate and cyclosporin, intravenous immunoglobulin, and TNFα-antagonists.

In another embodiment, the disease treated by the invention provided herein is experimental autoimmune uveitis (EAU). EAU is a T cell-mediated autoimmune disease that targets neural retina, uvea, and related tissues in the eye. EAU shares many clinical and immunological features with human autoimmune uveitis, and is induced by peripheral administration of uveitogenic peptide emulsified in Complete Freund's Adjuvant (CFA).

In another embodiment, the disease treated by the methods provided herein is primary biliary cirrhosis (PBC). PBC is an organ-specific autoimmune disease that predominantly affects women between 40-60 years of age. The prevalence reported among this group approaches 1 per 1,000. PBC is characterized by progressive destruction of intrahepatic biliary epithelial cells (IBEC) lining the small intrahepatic bile ducts. This leads to obstruction and interference with bile secretion, causing eventual cirrhosis. Association with other autoimmune diseases characterized by epithelium lining/secretory system damage has been reported, including Sjogren's Syndrome, CREST Syndrome, autoimmune thyroid disease and rheumatoid arthritis.

In another embodiment, the disease treated by the invention provided herein is celiac disease, also known as celiac sprue or gluten-sensitive enteropathy. Celiac disease is a disease that results from defective gastrointestinal absorption due to hypersensitivity to cereal grain storage proteins, including glutens or its product gliadin and glutenin, present in wheat, barley, and oats. The disease is caused by CD4 T cells that recognize gliadin as dietary antigen and these cells induce a T_{H}1-mediated chronic inflammatory response. Symptoms include diarrhea, weight loss, and steatorrhea, villous atrophy and malabsorption are seen. It may also be associated with dermatitis herpetifonns, a vesicular skin eruption. Celiac disease is associated with alleles DQB1*0302 and DQB1*0201 combined with DQA1*0301 and DQA1*0501. 95% of patients carry either DQB1*0201 or DQB1*0302. The strong HLA association is believed to be due to the capacity of DQ molecules encoded by DQB1*0201, DQA1*0501, DQB1*0302 and DQA1*0301 to efficiently present deaminated variants of glutamine-rich peptides derived from gliadin and glutenin.

In another embodiment, the use for treating an autoimmune disease in a subject further involves inhibiting the proliferation or function of T cells which are responsive to an autoantigen. The pathological process of autoimmune diseases and immune rejection is mediated by T cells. Upon binding to and recognition of an antigen, T cells proliferate, secrete cytokines and recruit additional inflammatory and cytotoxic cells to the site.

In yet another embodiment, the invention described herein for treating an autoimmune disease in a subject involve binding the random copolymer to a major histocompatibility complex class II protein which is associated with an autoimmune disease. The Class II MHC proteins are expressed predominantly on the surfaces of B lymphocytes and antigen presenting cells such as macrophages. These Class II MHC proteins have a peptide-binding cleft which is the site at which antigenic peptides are presented to T cells. When the present random copolymers bind to a major histocompatibility complex class II protein, those random copolymers can block or otherwise interfere with antigen presentation and/or T cell activation.

In a specific embodiments, the disease treated by the invention described herein comprises multiple sclerosis, type-I diabetes, Hashimoto's thyroiditis, Crohn's disease, rheumatoid arthritis, systemic lupus erythematosus (SLE), gastritis, autoimmune hepatitis, hemolytic anemia, autoimmune hemophilia, autoimmune lymphoproliferative syndrome (ALPS), autoimmune uveoretinitis, glomerulonephritis, Guillain-Barre syndrome, psoriasis, myasthenia gravis, autoimmune encephalomyelitis, Goodpasture's syndrome, Grave's disease, paraneoplastic pemphigus, autoimmune thrombocytopenic purpura, scleroderma with anti-collagen antibodies, mixed connective tissue disease, pernicious anemia, polymyositis, idiopathic Addison's disease, autoimmune-associated infertility, bullous pemphigoid, Sjogren's syndrome, idiopathic myxedema or colitis. In some embodiments, the subject is afflicted with more than one disease.

### V. Therapeutic compositions

The random copolymers of the present invention may be administered to the subject as a composition which comprises a pharmaceutically effective amount of copolymer and an acceptable carrier and/or excipients. A pharmaceutically acceptable carrier includes any solvents, dispersion media, or coatings that are physiologically compatible. The carrier is suitable for subcutaneous administration. One exemplary pharmaceutically acceptable carrier is physiological saline. Other pharmaceutically acceptable carriers and their formulations are well-known and generally described in, for example, Remington's Pharmaceutical Science (18th Ed, ed. Gennaro, Mack Publishing Co., Easton, PA, 1990). Various pharmaceutically acceptable excipients are well-known in the art and can be found in, for example, Handbook of Pharmaceutical Excipients (4th ed., Ed. Rowe et al. Pharmaceutical Press, Washington, D.C.). The composition can be formulated as a solution, microemulsion, liposome, capsule, tablet, or other suitable forms. The active component which comprises the copolymer may be coated in a material to protect it from inactivation by the environment prior to reaching the target site of action. The pharmaceutical compositions of the present invention are preferably sterile and non-pyrogenic at the time of delivery, and are preferably stable under the conditions of manufacture and storage.

In other embodiments of the present invention, the pharmaceutical compositions are regulated-release formulations. Copolymers of the present invention may be admixed with biologically compatible polymers or matrices which control the release rate of the copolymers into the immediate environment. Controlled or sustained release compositions include formulation in lipophilic depots (*e*.*g*., fatty acids, waxes, oils).

In some embodiments of the present invention, pharmaceutical compositions comprise random copolymers formulated with oil and emulsifier to form water-in-oil microparticles and/or emulsions. The oil may be any non-toxic hydrophobic material liquid at ambient temperature to about body temperature, such as edible vegetable oils including safflower oil, soybean oil, corn oil, and canola oil; or mineral oil. Chemically defined oil substance such as lauryl glycol may also be used. The emulsifier useful for this embodiment includes Span 20 (sorbitan monolaurate) and phosphatidylcholine. In some embodiments, a random copolymer composition is prepared as an aqueous solution and is prepared into an water-in-oil emulsion dispersed in 95 to 65% oil such as mineral oil, and 5 to 35% emulsifier such as Span 20. In another embodiment of the invention, the emulsion is formed with alum rather than with oil and emulsifier. These emulsions and microparticles reduce the speed of uptake of random copolymer, and achieves controlled antigen delivery.

In some embodiments, the pharmaceutical compositions also include additional therapeutically active agents. Such additional ingredient can be at least an additional random copolymer, such as a Copolymer 1 (YEAK, Copaxone^{™}) that binds to a different HLA molecule, an antibody which binds to an unwanted inflammatory molecule or cytokine such as interleukin-6, interleukin-8, granulocyte macrophage colony stimulating factor, and tumor necrosis factor-α; an enzyme inhibitor such as a protease inhibitor aprotinin or a cyclooxygenase inhibitor; an antibiotic such as amoxicillin, rifampicin, erythromycin; an antiviral agent such as acyclovir; a steroidal anti-inflammatory such as a glucocorticoid; a non-steroidal anti-inflammatory such as aspirin, ibuprofen, or acetaminophen; or a non-inflammatory cytokine such as interleukin-4 or interleukin-10. Other cytokines and growth factors such as interferon-β, tumor necrosis factors, antiangiogenic factors, erythropoietins, thrombopoietins, interleukins, maturation factors, chemotactic protein, and their variants and derivatives that retain similar physiological activities may also be used as an additional ingredient.

In some embodiments, the additional active therapeutically active agent is selected from the group consisting of anti-psoriasis creams, eye drops, nose drops, Sulfasalazine, glucocorticoids, propylthiouracil, methimazole, I¹³¹, insulin, IFN-β1a, IFN-β1b, glucocorticoids, ACTH, avonex, azathiopurine, cyclophosphamide, UV-B, PUVA, methotrexate, calcipitriol, cyclophosphamide, OKT3, FK-506, cyclosporin A, azathioprine, and mycophenolate mofetil.

Copolymers of the invention may also be used in combination with anti-obesity drugs. Anti-obesity drugs include P-3 agonists, CB-1 antagonists, appetite suppressants, such as, for example, sibutramine (Meridia), and lipase inhibitors, such as, for example, orlistat (Xenical). The subject copolymers may also be used in the invention in combination with drugs commonly used to treat lipid disorders in diabetic patients. Such drugs include, but are not limited to, HMG-CoA reductase inhibitors, nicotinic acid, bile acid sequestrants, and fibric acid derivatives. Polypeptides of the invention may also be used in combination with anti-hypertensive drugs, such as, for example, β-blockers, cathepsin S inhibitors and ACE inhibitors. Examples of β-blockers are: acebutolol, bisoprolol, esmolol, propanolol, atenolol, labetalol, carvedilol, and metoprolol. Examples of ACE inhibitors are: captopril, enalapril, lisinopril, benazepril, fosinopril, ramipril, quinapril, perindopril, trandolapril, and moexipril.

A kit may be provided comprising (i) a composition comprising the random copolymer and (ii) instructions for administering the composition to a subject in need thereof at intervals greater than 168 hours, for the treatment of an autoimmune disease. In one embodiment, the autoimmune disorder is multiple sclerosis. In another preferred embodiment, the random copolymer is formulated in dosages for administration of greater than about 168, 174, 180, 186, 192, 198, 204, 210, 216, 222, 228, 234, or 240 hours, or any intervening interval thereof. In another embodiment of the kits described herein, the instructions indicate that the random polymer is to be administered every about 168, 174, 180, 186, 192, 198, 204, 210, 216, 222, 228, 234, or 240 hours, or any interval in between. Kits may comprise additional components, such as packaging and one or more apparatuses for the administration of the copolymer, such as a hypodermic syringe.

In a specific embodiment, the autoimmune disease is selected from the group consisting of multiple sclerosis, type-I diabetes, Hashimoto's thyroiditis, Crohn's disease, rheumatoid arthritis, systemic lupus erythematosus (SLE), gastritis, autoimmune hepatitis, hemolytic anemia, autoimmune hemophilia, autoimmune lymphoproliferative syndrome (ALPS), autoimmune uveoretinitis, glomerulonephritis, Guillain-Barre syndrome, psoriasis, myasthenia gravis, autoimmune encephalomyelitis, Goodpasture's syndrome, Grave's disease, paraneoplastic pemphigus, autoimmune thrombocytopenic purpura, scleroderma with anti-collagen antibodies, mixed connective tissue disease, pernicious anemia, polymyositis, idiopathic Addison's disease, autoimmune-associated infertility, bullous pemphigoid, Sjogren's syndrome, idiopathic myxedema and colitis.

### VI. Treatment

The present invention provides compositions for use to treat a subject afflicted with or suspected of being afflicted with an autoimmune disease, by administering one or more random copolymers to the subject in a therapeutically effective amount. In particular, subcutaneous administration of a pharmaceutical composition comprising the random copolymer composition is provided in the invention. Subcutaneous injection induces more desired immune responses biased for T_{H}2 response, which is the basis for the tolerance for certain antigens.

In general, the treatment of the present invention, which is immunomodulation of the subject in need of such treatment, can be differentiated from vaccination. Successful vaccination is dependent on the immunogenicity of the vaccine being administered, which increases the titer of antibodies directly reactive to the antigens in the vaccine. In contrast, the random copolymers of the present invention are effective in treating diseases without inducing a high titer of antibodies against the copolymers themselves. As demonstrated by the Examples below, the effectiveness of the present invention does not depend on the antibody production against the copolymers, and therefore is fundamentally different from vaccination. Unlike vaccination, random copolymers of the present invention, administered according to the invention, induces tolerance toward the disease-related antigens, and more specifically, induces peripheral tolerance. Peripheral tolerance, in contract to central tolerance, has the advantage of being safer as a modulatory phenomenon.

In general, an embodiment of the invention is to administer a suitable dose of the therapeutic copolymer composition that will be the lowest effective dose to produce a therapeutic effect, for example, mitigating symptoms. The therapeutic copolymers are preferably administered at a dose per subject, which corresponds to a dose per day of at least about 2 mg, at least about 5 mg, at least about 10 mg, or at least about 20 mg as appropriate minimal starting dosages, or about x mg, wherein x is an integer between 1 and 20. In one embodiment described herein, a dose of about 0.01 to about 500 mg/kg can be administered. In general, the effective dosage of the compound of the present invention is about 50 to about 400 micrograms of the compound per kilogram of the subject per day. In one specific embodiment, the equivalent dosage per day, regardless of the frequency with which the doses are administered, is from about 5 to 100, or more preferably, from about 10 to 40, or more preferably about 20mg/day. In another specific embodiment, each individual dosage in the treatment regimen is from about 5 to 100, or more preferably from about 10 to 40, or more preferably about 20mg/dose.

However, it is understood by one skilled in the art that the dose of the composition of the invention will vary depending on the subject and upon the particular route of administration used. It is routine in the art to adjust the dosage to suit the individual subjects. Additionally, the effective amount may be based upon, among other things, the size of the compound, the biodegradability of the compound, the bioactivity of the compound and the bioavailability of the compound. If the compound does not degrade quickly, is bioavailable and highly active, a smaller amount will be required to be effective. The actual dosage suitable for a subject can easily be determined as a routine practice by one skilled in the art, for example a physician or a veterinarian given a general starting point. For example, the physician or veterinarian could start doses of the compound of the invention employed in the pharmaceutical composition at a level lower than that required in order to achieve the desired therapeutic effect, and increase the dosage with time until the desired effect is achieved. A physician or veterinarian may also refer to the recommendations for the administration of Copaxone™ as a general starting point.

In the context of the invention, the term "treatment regimen" is meant to encompass therapeutic, palliative and prophylactic modalities of administration of one or more compositions comprising one or more random copolymers. A particular treatment regimen may last for a period of time which will vary depending upon the nature of the particular disease or disorder, its severity and the overall condition of the patient.

Following treatment, the patient is monitored for changes in his/her condition and for alleviation of the symptoms of the disorder or disease state. The dosage of the polymer may either be increased in the event the patient does not respond significantly to current dosage levels, or the dose may be decreased if an alleviation of the symptoms of the disorder or disease state is observed, or if the disorder or disease state has been ablated, or if an unacceptable side effects are seen with the starting dosage.

In one embodiment, a therapeutically effective amount of the random copolymer is administered to the subject in a treatment regimen comprising intervals of at least 168, 174, 180, 186, 192, 198, 204, 210, 216, 222, 228, 234, or 240 hours, or the equivalent amount of days. In some embodiments, the agent is administered weekly. If two copolymers are administered to the subject, such copolymers may be administered at the same time, such as simultaneously, or essentially at the same time, such as in succession. Alternatively, their administration may be staggered.

As shown by the Examples below, treatment regimens with longer dosing intervals, consequently often with lower total exposure of copolymers, induce lower titers of antibodies against copolymers themselves, while still inducing desired protective effects. Such reduction of neutralizing antibodies are desirable because it is considered likely to help random copolymer compositions to retain its effectiveness without being neutralized, and it is associated with reduced risk of anaphylactic shocks, providing safer treatments of diseases. Longer interval regimens are also desirable because they strengthen the bias for T_{H}2 responses, which is considered to be the mode of action for the random copolymer therapies.

In one embodiment, the polymer is administered to be subject at least three times during a treatment regimen, such that there are at least two time intervals between administrations. These intervals maybe denoted I₁ and I₂. If the polymer is administered four times, then there would be an additional interval between the third and fourth administrations, I₃, such that the number of intervals for a given number "n" of administrations is n-I. Accordingly, the time intervals between administrations is greater than 168, 174, 180, 186, 192, 198, 204, 210, 216, 222, 228, 234, or 240 hours.

In yet another embodiment, the average time interval between administrations ((I₁ +I₂ +...+Iₙ₋₁)/n-1) is at least 168, 174, 180, 186, 192, 198, 204, 210, 216, 222, 228, 234, or 240 hours, or at least two weeks.

In another embodiment, the dosage regimen consists of two or more different Interval sets. For a dosing regimen comprising dosing of the random copolymer every week, the dose comprises up to about 500 mg/m², and for a dosing regimen comprising dosing of the random copolymer every two weeks or every month, up to 1.5 g/m² may be administered. The first part of the dosing regimen may be administered for up to 30 days, for example, 7, 14, 21, or 30 days. A subsequent second part of the dosing regimen with a different, longer interval administration with usually lower exposure (step-down dosage), administered weekly, every 14 days, or monthly may optionally follow, for example, at 500 mg/m² body surface area weekly, up to maximum of about 1.5 g/m² body surface area, continuing for 4 weeks up to two years, for example, 4, 6, 8, 12, 16, 26, 32, 40, 52, 63, 68, 78, or 104 weeks. Alternatively, if the disease goes into remission or generally improves, the dosage may be maintained or kept at lower than maximum amount, for example, at 140 mg/nr' body surface area weekly. If, during the step-down dosage regimen, the disease condition relapses, the first dosage regimen may be resumed until effect is seen, and the second dosing regimen may be implemented. This cycle may be repeated multiple times as necessary.

Any of the uses and means may be practiced using compositions and formulations described in this application.

In other embodiments of the invention, any of the uses of the invention may be practiced using sustained release formulation comprising a random copolymer. When administering a random copolymer of the invention using a sustained release formula, the overall exposure to the copolymer is generally lower than in bolus administration. In some embodiment of the invention, the dosing regimen uses sustained release formula, dosing the subject weekly, biweekly, or monthly so that the copolymer is released during the interval. For a dosing regimen comprising dosing of the random copolymer every week, the dose comprises up to about 140 mg/m², and for a dosing regimen comprising dosing of the random copolymer every two weeks or every month, up to 750 mg/m² may be administered. The first part of the dosing regimen may be administered for up to 30 days, for example, 7, 14, 21, or 30 days. A subsequent second part of the dosing regimen with a different, longer interval administration with usually lower exposure (step-down dosage), administered weekly, every 14 days, or monthly may optionally follow, for example, at 140 mg/m² body surface area weekly, up to maximum of about 1.5 g/m² body surface area, continuing for 4 weeks up to two years, for example, 4, 6, 8, 12, 16, 26, 32, 40, 52, 63, 68, 78, or 104 weeks. Alternatively, if the disease goes into remission or generally improves, the dosage may be maintained or kept at lower than maximum amount, for example, at 140 mg/m² body surface area weekly. If, during the step-down dosage regimen, the disease condition relapses, the first dosage regimen may be resumed until effect is seen, and the second dosing regimen may be implemented. This cycle may be repeated multiple times as necessary.

In any of the exemplary embodiments described above, the volume of the each dosage form is preferably 0.1ml to 5ml.

In the invention, the route of administration is subcutaneous. In the invention, the random copolymer is administered subcutaneously.

In an embodiment of the present invention the administration of the copolymers of the present invention is in a sustained release form. Such uses comprise implanting a sustained-release capsule or a coated implantable medical device so that a therapeutically effective dose of the copolymer of the present invention is delivered at defined time intervals to a subject of such a treatment. The compounds and/or agents of the invention may be delivered via a capsule which allows regulated-release of the random copolymer over a period of time. Controlled or sustained· release compositions include formulation in lipophilic depots (e.g., fatty acids, waxes, oils). Also comprehended by the invention are particulate compositions coated with polymers (e.g., poloxamers or poloxamines). In certain embodiments, a source of a copolymer is stereotactically provided within or proximate to the area of autoimmune attack, for example, near the pancreas for the treatment of IDDM.

In certain embodiments, the inventions described herein allow continuous treatment of autoimmune diseases by a sustained-release carrier such as transdermal patches, implantable medical devices coated with sustained-release formulations, or implantable or injectable pharmaceutical formulation suitable for sustained-release of the active components.

In a specific embodiment, the random copolymers are formulated for a long-lasting therapeutic affect such that a therapeutic effect in treating the disease is observed when the random copolymers are administered to the subject at time intervals of at least 168, 174, 180, 186, 192, 198, 204, 210, 216, 222, 228, 234, or 240 hours between administrations.

The present invention also provides for prophylactically treating a subject at risk of developing *e*.*g*., an autoimmune disease by administering a random copolymer. A subject at risk is identified by, for example, determining the genetic susceptibility to an autoimmune disease by testing for alleles of HLA that are associated with such autoimmune disease, and/or based on familial history, or other genetic markers that correlate with such autoimmune disease. Such prophylactic treatment may additionally comprise a second copolymer that binds to a second HLA molecule associated with the autoimmune disease to be treated. The second HLA molecule may be a HLA-DQ or HLA-DR molecule. Preferably, the autoimmune disease to be prophylactically treated is IDDM or celiac disease.

In other embodiments of the invention described herein, additional therapeutically active agents are administered to the subject. In one embodiment, compositions comprising additional therapeutic agents(s) are administered to the subject as separate compositions from those comprising the random polymer. For example, a subject may be administered a composition comprising a random copolymer subcutaneously while a composition comprising another therapeutic agent may be administered orally. The additional therapeutically active agents may treat the same disease as the random copolymer, a related disease, or may be intended to treat an undesirable side effect of administration of the copolymer, such as to reduce swelling at a site of intradermal injection.

Additional therapeutically active agents which may be administered to the subject include copolymers which bind to a second HLA molecule associated with the disease, such as Copaxone™; an antibody, an enzyme inhibitor, an antibacterial agent, an antiviral agent, a steroid, a nonsteroidal anti-inflammatory agent, an antimetabolite, a cytokine, or a soluble cytokine receptor. The second HLA molecule may he an HLA-DQ molecule or an HLA-DR molecule. The enzyme inhibitor may be a protease inhibitor or a cyclooxygenase inhibitor. The additional agent may be added as a part of the pharmaceutical composition, or may be administered concomitantly or within a time period when the physiological effect of the additional agent overlaps with the physiological effect of the copolymer of the present invention. More particularly, an additional agent may be administered concomitantly or one week, several days, 24 hours, 8 hours, or immediately before the administration of the copolymer. Alternatively, an additional agent may be administered one week, several days, 24 hours, 8 hours, or immediately after the administration of the copolymer.

An improvement in the symptoms of a subject afflicted with multiple sclerosis (MS) as a result of administration of the random copolymer may be noted by a decrease in frequency of recurrences of episodes of MS, by decrease in severity of symptoms, and by elimination of recurrent episodes for a period of time after the start of administration. A therapeutically effective dosage preferably reduces symptoms and frequency of recurrences by at least about 20%, for example, by at least about 40%, by at least about 60%, and by at least about 80%, or by about 100% elimination of one or more symptoms, or elimination of recurrences of the autoimmune disease, relative to untreated subjects. The period of time can be at least about one month, at least about six months, or at least about one year.

An improvement in the symptoms of a subject afflicted with arthritis or any other autoimmune disorder which results in inflammation of the joints may be noted by a reduction in edema of one or more joints, by a reduction in inflammation in one or more joints, or by an increase in mobility in one or more joints. A therapeutically effective dosage preferably reduces joint inflammation and edema and improves mobility by at least about 20%, more preferably by at least about 40%, even more preferably by at least about 60%, and even still more preferably by at least about 80%, relative to untreated subjects.

The practice of the present invention will employ, where appropriate and unless otherwise indicated, conventional techniques of cell biology, cell culture, molecular biology, transgenic biology, microbiology, virology, recombinant DNA, and immunology, which are within the skill of the art. Such techniques are described in the literature. See, for example, Molecular Cloning: A Laboratory Manual, 3rd Ed., ed. by Sambrook and Russell (Cold Spring Harbor Laboratory Press: 2001); the treatise, Methods In Enzymology (Academic Press, Inc., N.Y.); Using Antibodies, Second Edition by Harlow and Lane, Cold Spring Harbor Press, New York, 1999; Current Protocols in Cell Biology, ed. by Bonifacino, Dasso, Lippincott-Schwartz, Harford, and Yamada, John Wiley and Sons, Inc., New York, 1999; and PCR Protocols, ed. by Bartlett et al., Humana Press, 2003.

### VII. Examples

### Example 1. Production of antibodies against random copolymers and a disease-associated antigen peptide

PLP(139-151) peptide is the major immunogenic determinant recognized by CD4⁺ T_{H}1 cells which in turn drive EAE development in SJL mice. When injected with pertussis toxin, PLP (139-151) peptide causes MS-like symptoms in the SJL mice. In the absence of the pertussis toxin, injected animals develop only mild and transient disease. The ability of random copolymer compositions to protect the animals from the effect of PLP injection was evaluated in the course of daily and weekly dosing of the animals after their exposure to PLP (139-151) peptide. Antibody isotypes were also examined. CD4 T cells can be divided into at least two different subsets depending on the pattern of their cytokine production. T_{H}1 cells preferentially produce IL-2 and IFN-γ, activate macrophages, and stimulate production of the Ig subclasses IgG2a and IgG3 in mice and IgG1 and IgG3 in humans. In contrast, the signature cytokines of T_{H}2 cells are IL-4, IL-5, and IL-13, which provide potent B cell help and induce isotype switching to IgE and IgG1 in mice or to IgE, IgG2, and IgG4 in humans. Therefore, mouse IgG1 and IgG2b, generally associated with T_{H}2 response, and mouse IgG2a, markers of T_{H}1 immunity, were measured.

Mice (SJL, female) were immunized on day 1 with 100µg of PLP(139-151) peptide in Complete Freund's adjuvant. The same day, the animals received an intravenous injection of 200 ng of pertussis toxin. On day 3, the same IV injection was repeated. Treatment with Copaxone^{™} (YEAK) or Co-14 (YFAK), 7.5mg/kg, daily and weekly was started on day 6 and continued daily until day 36. On day 37, individual sera were collected and antibody response against PLP (139-151) peptide, Co-14 (YFAK), and Copaxone^{™} were measured using standard ELISA with anti-mouse total Ig, IgG1, IgG2a or IgG2b as secondary antibody.

During the course of the experiment, disease severity was measured using a standard scoring system between 0 (no disease) and 5 (moribund), and body weight of a mouse was recorded as another measure of disease state. The mortality rate of the animals was recorded daily.

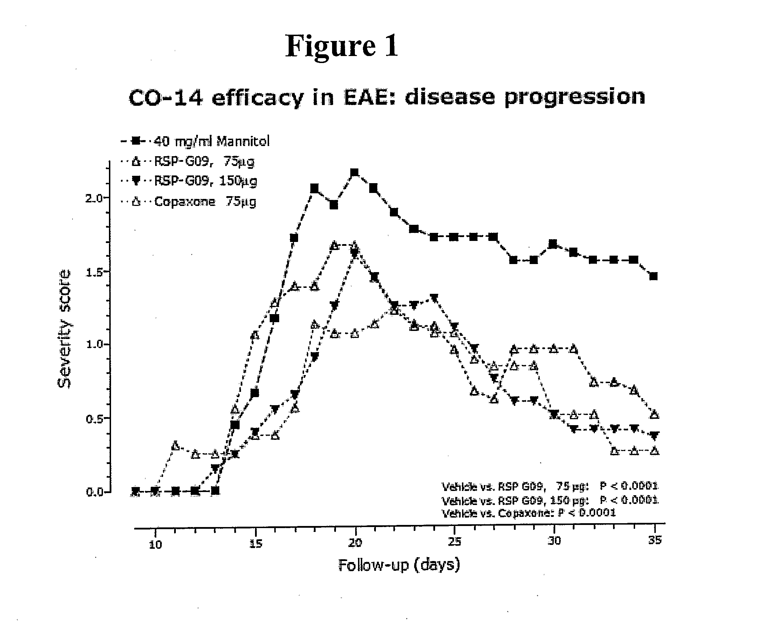

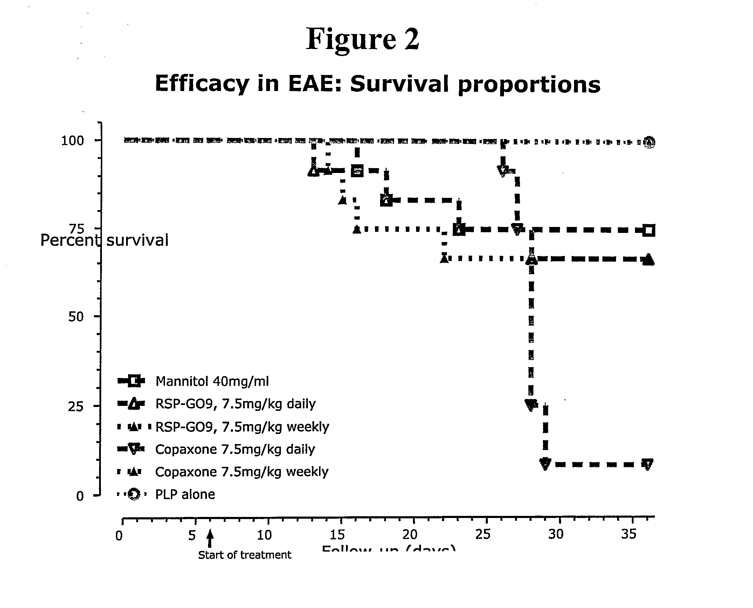

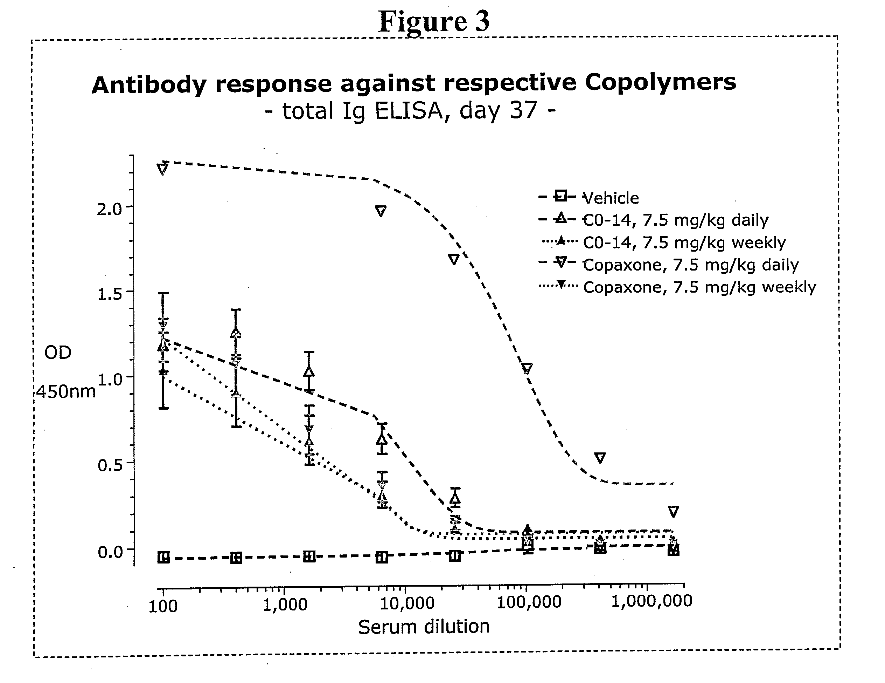

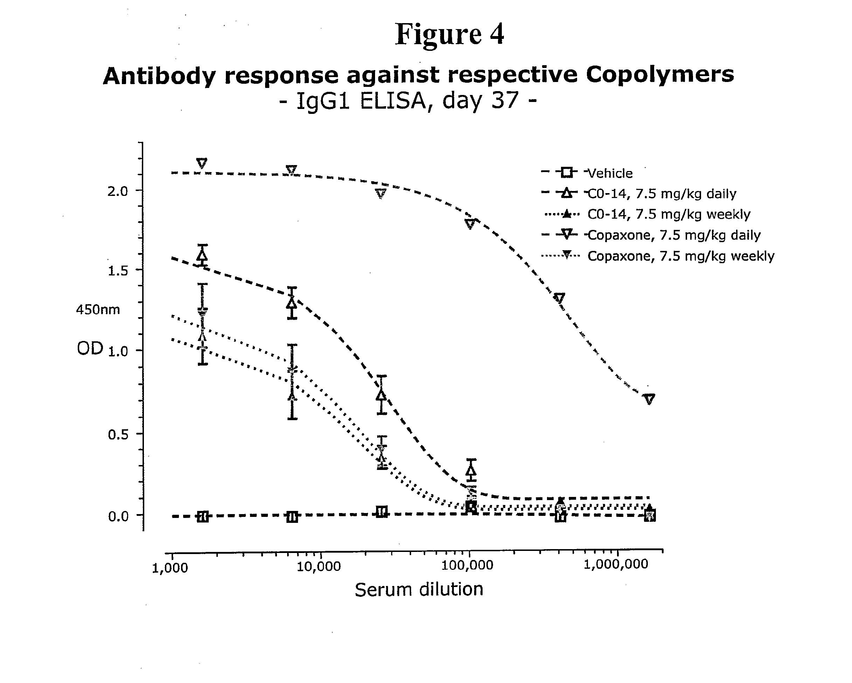

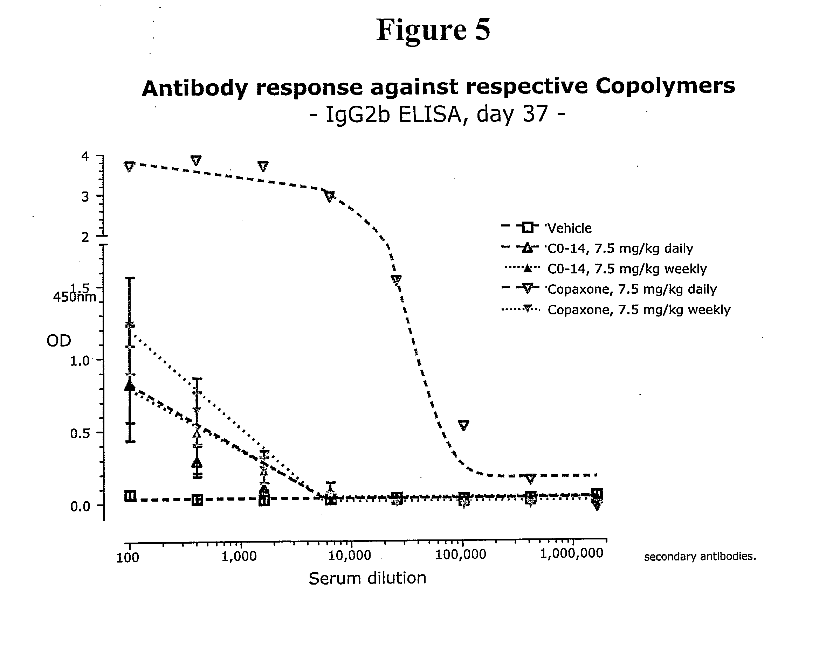

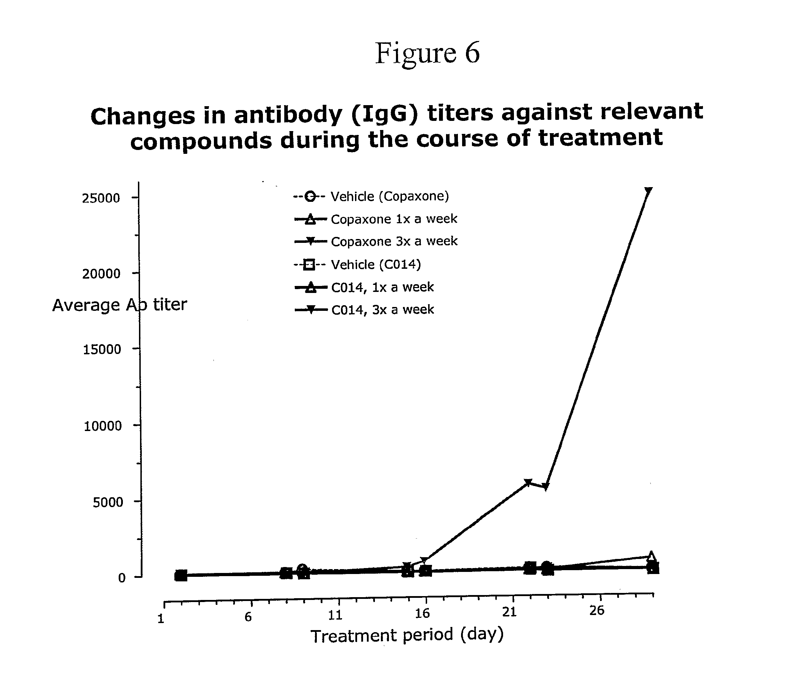

Although daily dosing of Copaxone^{™} was effective in reducing the severity of the disease compared to mannitol dosing alone (Figure 1), majority of the mice treated with daily dose of Copaxone^{™} died suddenly after about 3 weeks of treatment (Figure 2). As shown in Figure 3, daily dosing of Copaxone^{™} induced a large amount of antibodies in the surviving injected mice. In contrast, weekly dosing with Copaxone^{™}, and daily and weekly dosing with Co-14 (YFAK), resulted in much lower antibody titers. The immune response was predominantly IgGl+IgG2b (i.e., predominantly T_{H}2) responses, and a much lower IgG2a (i.e. T_{H}1) response was seen. The few surviving mice in Copaxone^{™} daily group had large IgG1 and IgG2b response against compound (Figures 4 and 5), raising the possibility that the cause of death in Copaxone^{™} daily dosed mice is likely to be anaphylaxis. In contrast, weekly dosing with Copaxone^{™}, and daily and weekly dosing with Co-14 (YFAK), which showed a much lower antibody titers, prevented anaphylactic shock and increased efficacy. Another example of antibody titers is shown in Figure 6, where Copaxone^{™} and Co-14 (YFAK) were administered either once a week or 3 times a week. Copaxone^{™}, when administered 3 times a week, induces production of large amount of antibodies directed against it, whereas weekly dosing of Copaxone^{™} and dosing of Co- 14, either weekly or three times a week, do not induce appreciative amount of antibodies against the respective copolymers.

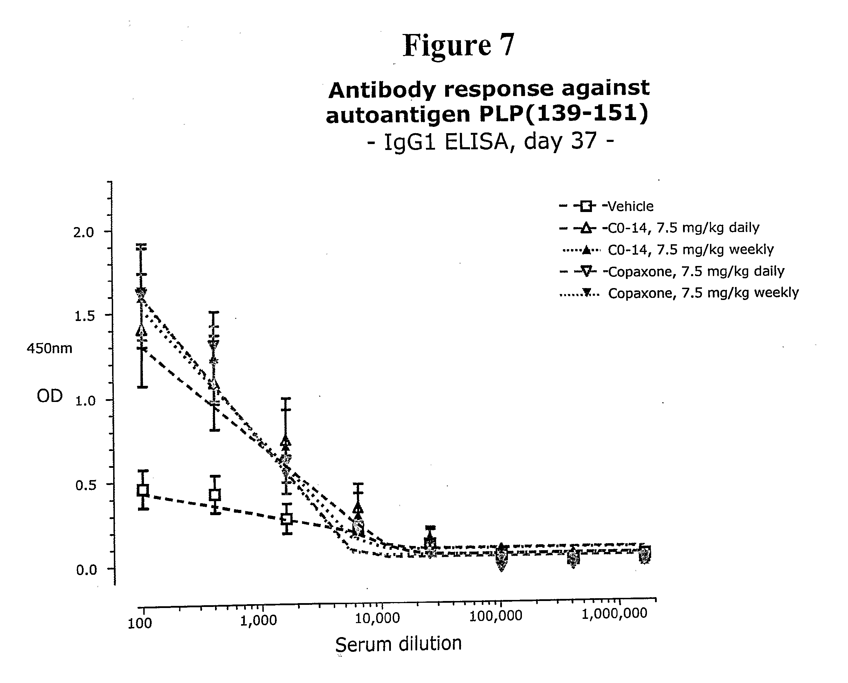

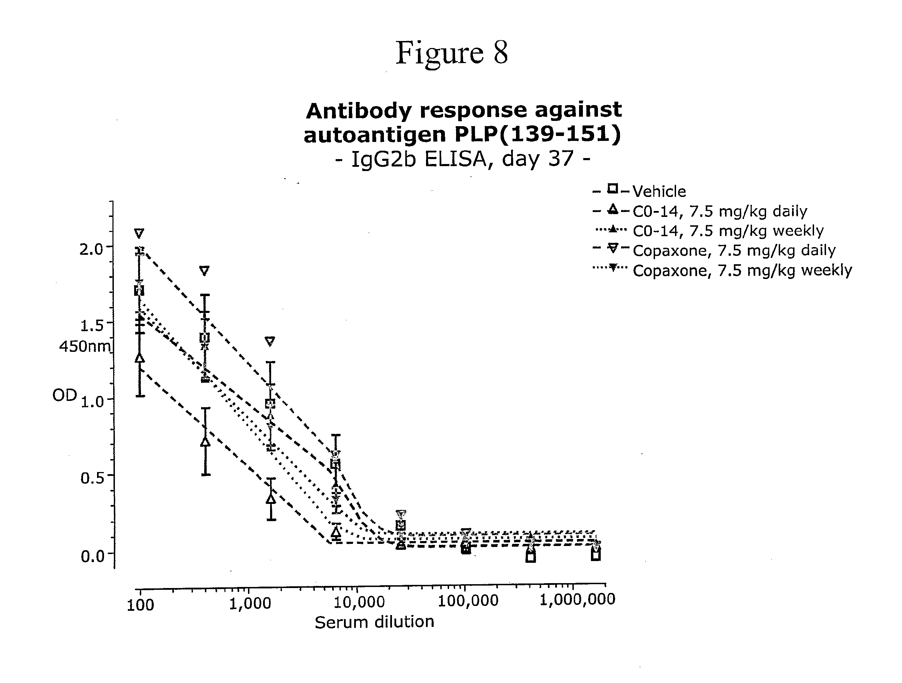

When antibody titers for PLP (139-151) peptide were measured, both Copaxone^{™} and Co-14 (YFAK), regardless of dosing interval, induced similar, small increases in amounts of IgG1 formation against PLP (139-151) peptide (Figure 7) compared to dosing with vehicle alone. The titers of IgG2b against PLP (139-151) was also not significantly affected (Figure 8). These results show that the protective effect of Copaxone^{™} or Co-14 (YFAK) is not exerted through modulation of antibody amounts against PLP (139-151) peptide.

### Example 2. T cell response to random copolymers

The T_{H}1 and T_{H}2 profiles of mice injected with 5 µg Copaxone^{™} or Co-14 (YFAK) three times a week or on weekly bases, up to day 22 of the treatment. On day 2, 8, 9, 15, 16, 22, 23, 29, spleens were collected and splenocytes were isolated. 400,000 cells per well of splenocytes were restimulated with various concentrations (0.8, 4, or 20 µg /ml) of Co-14 (YFAK) for three days. On day 3 of the cell culture, the cells were transferred onto ELISPOT (enzyme-linked immunospot assay) plates, coated with either IFN-γ (interferon gamma) or IL-13 (interleukin 13). The T cell response is examined by measuring the IFN γ production (a T_{H}1 cytokine) and IL-13 production (a T_{H}2 cytokine). The degree of T cell stimulation is also examined by measuring the proliferation of the cells shown as tritiated thymidine intake.

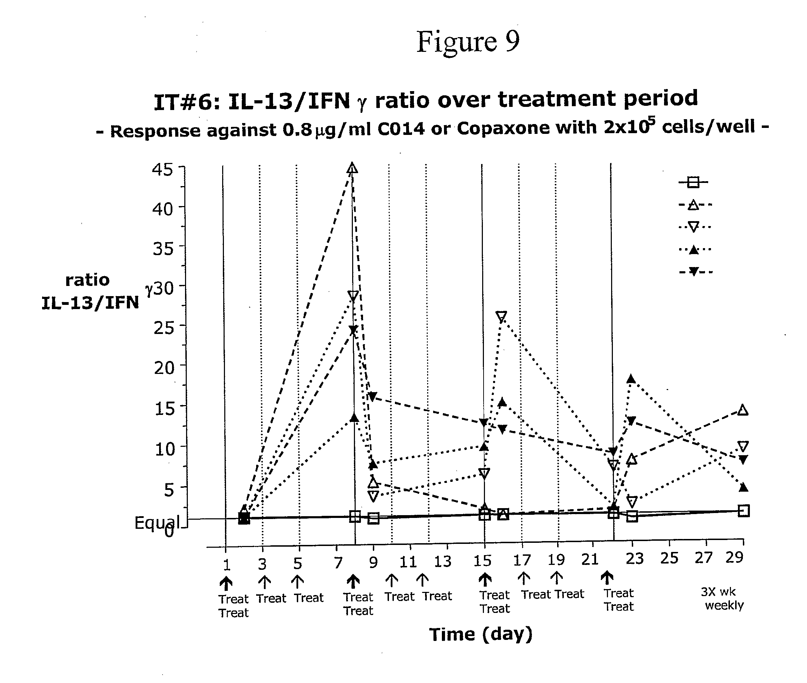

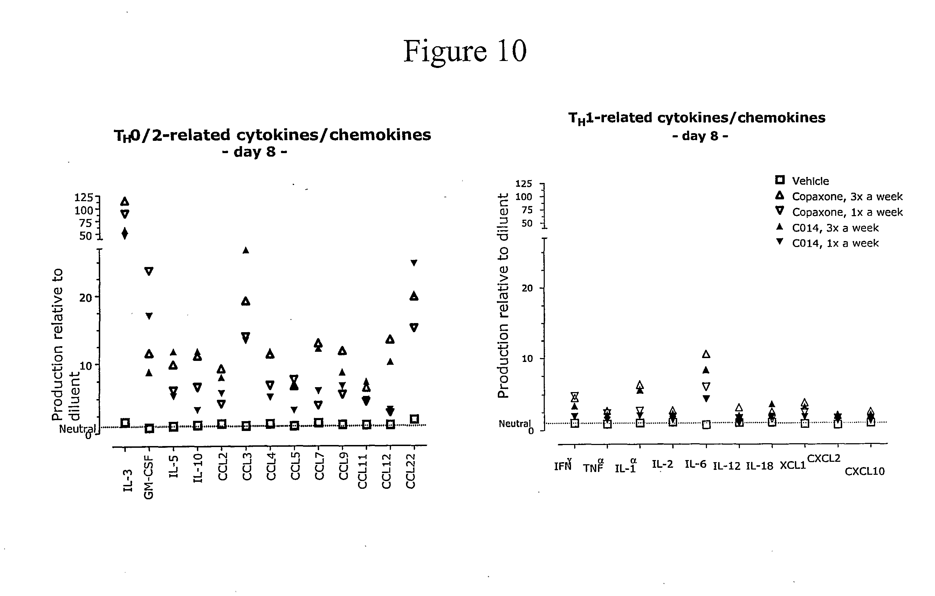

A burst of response was seen in the first week of dosing, followed by a decreased but sustained response. As seen in Figure 9, the response is T_{H}2 biased, with the IL-13 production induced more strongly than the IFN- γ at all times in cells treated with either Copaxone^{™} or Co-14 (YFAK). The T_{H}2 bias is further confirmed by the amount of 23 cytokines and chemokines, as seen in Figure 10.

## Claims

1. A random copolymer composition for use in treating an autoimmune disease mediated by T_{H}1 cells, wherein the random copolymer composition comprises random copolymers consisting of amino acid residues YFAK (L-tyrosine, L-phenylalanine, L-alanine and L-lysine) in a molar output ratio of about 1.0: 1.2: 20.0<30.0: 6.0 respectively, wherein the variability in the output ratio comprises a range of 10% between the different amino acid residues, and the length of the copolymer is between 35 and 75 amino acid residues, wherein the composition is to be delivered subcutaneously to a subject in two or more doses, each dose separated by a time interval of at least 7 days, thereby inducing a T_{H}2 biased immune response.

2. The random copolymer composition for use according to claim 1, wherein the random copolymers consist of amino acid residues YFAK (L-tyrosine, L-phenylalanine, L-alanine and L-lysine) in a molar output ratio of about 1.0: 1.2: 22.0<30.0: 6.0 respectively.

3. The random copolymer composition for use according to claim 1 or 2, wherein the random copolymers consist of amino acid residues YFAK (L-tyrosine, L-phenylalanine, L-alanine and L-lysine) in a molar output ratio of about 1.0: 1.2: X_{A}: 6.0 respectively, wherein X_{A} is 22.0 or 24.0.

4. The random copolymer composition for use according to any one of claims 1 to 3, wherein the ratio of alanine increases with the length of copolymer.

5. The random copolymer composition for use according to any one of claims 1 to 4, wherein the composition is for administration to a mammal.

6. The random copolymer composition for use according to claim 5, wherein the mammal is a human.

7. The random copolymer composition for use according to any one of claims 1 to 6, wherein the amount of random copolymer composition consists of between 10mg and 30mg.

8. The random copolymer composition for use according to claim 7, wherein the amount of random copolymer composition consists of between 15mg and 25mg.

9. The random copolymer composition for use according to claim 8, wherein the amount of random copolymer composition is about 20mg.

10. The random copolymer composition for use according to any one of claims 1 to 6, wherein the amount of random copolymer composition is less than 20mg.

11. The random copolymer composition for use according to claim 1, wherein the disease is selected from: multiple sclerosis, type-I diabetes, Hashimoto's thyroiditis, Crohn's disease, rheumatoid arthritis, systemic lupus erythematosus (SLE), gastritis, autoimmune hepatitis, hemolytic anemia, autoimmune hemophilia, autoimmune lymphoproliferative syndrome (ALPS), autoimmune uveoretinitis, glomerulonephritis, Guillain-Barré syndrome, psoriasis, myasthenia gravis, autoimmune encephalomyelitis, Goodpasture's syndrome, Grave's disease, paraneoplastic pemphigus, autoimmune thrombocytopenic purpura, scleroderma with anti-collagen antibodies, mixed connective tissue disease, pernicious anemia, polymyositis, idiopathic Addison's disease, autoimmune-associated infertility, bullous pemphigoid, Sjogren's syndrome, idiopathic myxedema and colitis.

12. The random copolymer composition for use according to claim 11, wherein the disease is multiple sclerosis.

13. The random copolymer composition for use according to claim 12, wherein the disease is relapsing remitting multiple sclerosis.

14. The random copolymer composition for use according to claim 1, wherein the random copolymer is for administration via devices designed to deliver the random copolymer continuously.

15. The random copolymer composition for use according to claim 1, wherein the composition is a sustained-release formulation comprising a random copolymer.

16. The random copolymer composition for use according to claim 1, wherein the composition is for administration with an additional therapeutically active agent.

17. The random copolymer composition for use according to claim 16, wherein the additional agent is one or more random copolymers.

18. The random copolymer composition for use according to claim 16, wherein the agent is an anti-inflammatory agent.

19. The random copolymer composition for use according to claim 15, wherein the composition is a sustained release formulation for delivery of an effective amount of the random copolymer composition over a period of at least 3 days.

## Patentansprüche

1. Statistische Copolymerzusammensetzung für den Gebrauch beim Behandeln einer durch T_{H}1-Zellen vermittelten Autoimmunkrankheit, wobei die statistische Copolymerzusammensetzung statistische Copolymere umfasst, die aus Aminosäureresten YFAK (L-Tyrosin, L-Phenylalanin, L-Alanin und L-Lysin) in einem molaren Ausgangsverhältnis von etwa 1,0:1,2:20,0<30,0:6,0 bestehen, wobei die Variabilität im Ausgangsverhältnis einen Bereich von 10 % zwischen den verschiedenen Aminosäureresten umfasst und die Länge des Copolymers zwischen 35 und 75 Aminosäureresten beträgt, wobei die Zusammensetzung einem Subjekt in zwei oder mehr Dosen subkutan verabreicht werden soll, wobei jede Dosis durch einen Zeitraum von wenigstens 7 Tagen von anderen getrennt ist, um somit eine von T_{H}2 beeinflusste Immunantwort anzuregen.

2. Statistische Copolymerzusammensetzung zum Gebrauch nach Anspruch 1, wobei die statistischen Copolymere aus Aminosäureresten YFAK (L-Tyrosin, L-Phenylalanin, L-Alanin und L-Lysin) in einem molaren Ausgangsverhältnis von etwa 1,0:1,2:22,0<30,0:6,0 bestehen.

3. Statistische Copolymerzusammensetzung zum Gebrauch nach Anspruch 1 oder 2, wobei die statistischen Copolymere aus Aminosäureresten YFAK (L-Tyrosin, L-Phenylalanin, L-Alanin und L-Lysin) in einem molaren Ausgangsverhältnis von etwa 1,0:1,2:X_{A}:6,0 bestehen, wobei X_{A} 22,0 oder 24,0 ist.

4. Statistische Copolymerzusammensetzung zum Gebrauch nach einem der Ansprüche 1 bis 3, wobei der Alaninanteil mit der Länge des Copolymers ansteigt.

5. Statistische Copolymerzusammensetzung zum Gebrauch nach einem der Ansprüche 1 bis 4, wobei die Zusammensetzung zum Verabreichen an ein Säugetier gedacht ist.

6. Statistische Copolymerzusammensetzung zum Gebrauch nach Anspruch 5, wobei das Säugetier ein Mensch ist.

7. Statistische Copolymerzusammensetzung zum Gebrauch nach einem der Ansprüche 1 bis 6, wobei die Menge der statistischen Copolymerzusammensetzung zwischen 10 mg und 30 mg beträgt.

8. Statistische Copolymerzusammensetzung zum Gebrauch nach Anspruch 7, wobei die Menge der statistischen Copolymerzusammensetzung zwischen 15 mg und 25 mg beträgt.

9. Statistische Copolymerzusammensetzung zum Gebrauch nach Anspruch 8, wobei die Menge der statistischen Copolymerzusammensetzung etwa 20 mg beträgt.

10. Statistische Copolymerzusammensetzung zum Gebrauch nach einem der Ansprüche 1 bis 6, wobei die Menge der statistischen Copolymerzusammensetzung weniger als 20 mg beträgt.

11. Statistische Copolymerzusammensetzung zum Gebrauch nach Anspruch 1, wobei die Krankheit ausgewählt ist aus: multipler Sklerose, Typ-I-Diabetes, Hashimoto-Thyreoiditis, Morbus Crohn, rheumatoider Arthritis, systemischem Lupus erythematodes (SLE), Gastritis, autoimmuner Hepatitis, hämolytischer Anämie, autoimmuner Hämophilie, autoimmunem lymphoproliferativem Syndrom (ALPS), autoimmuner Uveoretinitis, Glomerulonephritis, Guillain-Barré-Syndrom, Psoriasis, Myasthenia gravis, autoimmuner Enzephalomyelitis, Goodpasture-Syndrom, Morbus Basedow, paraneoplastischem Pemphigus, autoimmuner thrombozytopenischer Purpura, Sklerodermie mit Anti-Kollagen-Antikörpern, Sharp-Syndrom, perniziöser Anämie, Polymyositis, idiopathischer Addisonscher Krankheit, mit Autoimmunstörungen verbundener Unfruchtbarkeit, bullösem Pemphigoid, Sjögren-Syndrom, idiopathischem Myxödem und Kolitis.

12. Statistische Copolymerzusammensetzung zum Gebrauch nach Anspruch 11, wobei die Krankheit multiple Sklerose ist.

13. Statistische Copolymerzusammensetzung zum Gebrauch nach Anspruch 12, wobei die Krankheit schubförmige remittierende multiple Sklerose ist.

14. Statistische Copolymerzusammensetzung zum Gebrauch nach Anspruch 1, wobei das statistische Copolymer zum Verabreichen über Vorrichtungen vorgesehen ist, die gestaltet sind, das statistische Copolymer dauerhaft zu verabreichen.

15. Statistische Copolymerzusammensetzung zum Gebrauch nach Anspruch 1, wobei die Zusammensetzung eine Formulierung mit einem statistischen Copolymer zur anhaltenden Freisetzung ist.

16. Statistische Copolymerzusammensetzung zum Gebrauch nach Anspruch 1, wobei die Zusammensetzung zum Verabreichen mit einem zusätzlichen therapeutischen Wirkstoff vorgesehen ist.

17. Statistische Copolymerzusammensetzung zum Gebrauch nach Anspruch 16, wobei der zusätzliche Stoff ein oder mehrere statistische Copolymere sind.

18. Statistische Copolymerzusammensetzung zum Gebrauch nach Anspruch 16, wobei der Stoff ein entzündungshemmendes Mittel ist.

19. Statistische Copolymerzusammensetzung zum Gebrauch nach Anspruch 15, wobei die Zusammensetzung eine Formulierung zur anhaltenden Freisetzung zur Verabreichung einer wirksamen Menge des statistischen Copolymers über einen Zeitraum von wenigstens 3 Tagen ist.

## Revendications

1. Composition de copolymères aléatoires pour une utilisation dans le traitement d'une maladie auto-immune médiée par les cellules T_{H}1, la composition de copolymères aléatoires comprenant des copolymères aléatoires constitués de résidus d'acides aminés YFAK (L-tyrosine, L-phénylalanine, L-alanine et L-lysine) dans un rapport de rendement molaire d'environ 1,0:1,2:20,0<30,0:6,0 respectivement, où la variabilité du rapport de rendement comprend une plage de 10 % entre les différents résidus d'acides aminés, et la longueur du copolymère est comprise entre 35 et 75 résidus d'acides aminés, où la composition est destinée à être administrée par voie sous-cutanée à un patient en deux doses ou plus, chaque dose étant séparée par un intervalle de temps d'au moins 7 jours, provoquant ainsi une réponse immunitaire biaisée vers T_{H}2.

2. Composition de copolymères aléatoires pour une utilisation selon la revendication 1, dans laquelle les copolymères aléatoires sont constitués de résidus d'acides aminés YFAK (L-tyrosine, L-phénylalanine, L-alanine et L-lysine) dans un rapport de rendement molaire d'environ 1,0:1,2:22,0<30,0:6,0 respectivement.

3. Composition de copolymères aléatoires pour une utilisation selon la revendication 1 ou 2, dans laquelle les copolymères aléatoires sont constitués de résidus d'acides aminés YFAK (L-tyrosine, L-phénylalanine, L-alanine et L-lysine) dans un rapport de rendement molaire d'environ 1,0:1,2:X_{A}:6,0 respectivement, où X_{A} vaut 22,0 ou 24,0.

4. Composition de copolymères aléatoires pour une utilisation selon l'une quelconque des revendications 1 à 3, dans laquelle le rapport de l'alanine augmente avec la longueur du copolymère.

5. Composition de copolymères aléatoires pour une utilisation selon l'une quelconque des revendications 1 à 4, la composition étant destinée à être administrée à un mammifère.

6. Composition de copolymères aléatoires pour une utilisation selon la revendication 5, dans laquelle le mammifère est un être humain.

7. Composition de copolymères aléatoires pour une utilisation selon l'une quelconque des revendications 1 à 6, dans laquelle la quantité de composition de copolymères aléatoires est comprise entre 10 mg et 30 mg.

8. Composition de copolymères aléatoires pour une utilisation selon la revendication 7, dans laquelle la quantité de composition de copolymères aléatoires est comprise entre 15 mg et 25 mg.

9. Composition de copolymères aléatoires pour une utilisation selon la revendication 8, dans laquelle la quantité de composition de copolymères aléatoires est d'environ 20 mg.

10. Composition de copolymères aléatoires pour une utilisation selon l'une quelconque des revendications 1 à 6, dans laquelle la quantité de composition de copolymères aléatoires est inférieure à 20 mg.

11. Composition de copolymères aléatoires pour une utilisation selon la revendication 1, dans laquelle la maladie est choisie parmi : la sclérose en plaques, le diabète de type I, la thyroïdite de Hashimoto, la maladie de Crohn, la polyarthrite rhumatoïde, le lupus érythémateux disséminé (LED), la gastrite, l'hépatite auto-immune, l'anémie hémolytique, l'hémophilie auto-immune, le syndrome lymphoprolifératif auto-immun (SLAI), l'uvéorétinite auto-immune, la glomérulonéphrite, le syndrome de Guillain-Barré, le psoriasis, la myasthénie grave, l'encéphalomyélite auto-immune, le syndrome de Goodpasture, la maladie de Grave, le pemphigus paranéoplasique, le purpura thrombopénique auto-immun, la sclérodermie avec anticorps anti-collagène, la connectivite mixte, l'anémie pernicieuse, la polymyosite, la maladie d'Addison idiopathique, la stérilité auto-immune, la pemphigoïde bulleuse, le syndrome de Sjögren, le myxoedème idiopathique et la colite.

12. Composition de copolymères aléatoires pour une utilisation selon la revendication 11, dans laquelle la maladie est la sclérose en plaques.

13. Composition de copolymères aléatoires pour une utilisation selon la revendication 12, dans laquelle la maladie est la sclérose en plaques cyclique.

14. Composition de copolymères aléatoires pour une utilisation selon la revendication 1, dans laquelle le copolymère aléatoire est destiné à être administré par le biais de dispositifs conçus pour libérer le copolymère aléatoire de façon continue.

15. Composition de copolymères aléatoires pour une utilisation selon la revendication 1, la composition étant une formulation à libération prolongée comprenant un copolymère aléatoire.

16. Composition de copolymères aléatoires pour une utilisation selon la revendication 1, la composition étant destinée à être administrée avec un agent thérapeutiquement actif supplémentaire.

17. Composition de copolymères aléatoires pour une utilisation selon la revendication 16, dans laquelle l'agent supplémentaire est un ou plusieurs copolymères aléatoires.

18. Composition de copolymères aléatoires pour une utilisation selon la revendication 16, dans laquelle l'agent est un agent anti-inflammatoire.

19. Composition de copolymères aléatoires pour une utilisation selon la revendication 15, la composition étant une formulation à libération prolongée destinée à libérer une quantité efficace de la composition de copolymères aléatoires sur une période d'au moins 3 jours.
